# EUROPEAN PATENT APPLICATION

(11) **EP 4 505 977 A1**
(43) Date of publication of application: **12.02.2025**
(21) Application number: 22939480.4
(22) Date of filing: 18.05.2022
(51) Int. Cl.: A61F 2/24

(54) **VALVE CONVEYING APPARATUS**

(30) Priority: 25.04.2022 CN 202210444121
(71) Applicant: Kingstronbio (Changshu) Co., Ltd., Changshu Jiangsu 215500 (CN)
(72) Inventor: ZHANG, Bo, Changshu, Jiangsu 215500 (CN); ZHONG, Shengping, Changshu, Jiangsu 215500 (CN); MENG, Chunwang, Changshu, Jiangsu 215500 (CN)
(74) Representative: Wu, Ting
(86) International application number: PCT/CN2022/093492
(87) International publication number: WO 2023/206646

(57) **Abstract**

Provided is a valve delivery device, including a bending adjustment tube (1), a withdrawing assembly (2), a traction wire (4), a bending adjustment wheel (3) and a stabilizing tube (5). The withdrawing assembly (2) is connected to the bending adjustment tube (1). The bending adjustment wheel (3) is provided with a traction block (31). One end of the traction wire (4) is connected to the withdrawing assembly (2), and the other end is connected to the traction block (31). The stabilizing tube (5) sleeves the bending adjustment tube (1) and is arranged between the withdrawing assembly (2) and the bending adjustment wheel (3). The bending adjustment wheel (3) is configured for adjusting the position of the traction block (31), and the traction block (31) drives the withdrawing assembly (2) to move by means of the traction wire (4) so as to adjust the bending degree of the bending adjustment tube (1), and the stabilizing tube (5) is configured for limiting the bending position of the bending adjustment tube (1), thereby improving the control precision of the bending shape of the valve delivery device.

## Description

### TECHNICAL FIELD

The present disclosure relates to the technical field of medical devices, and in particular, to a valve delivery device.

### BACKGROUND

With the continuous development of science and technology, the emergence of artificial valves has addressed many medical problems. For example, an implantable artificial heart valve is used to replace the heart valve by being implanted into the heart to treat heart diseases. The implantation of the heart valve needs an a valve delivery device. The valve delivery device delivers the artificial valve outside the body through blood vessels to a designated position and releases it. However, due to the complex structure of the physiological valve, there is an angular deviation between an access path during valve intervention and the valve axis. This requires that the valve delivery device should have a certain angle adjustment ability to achieve coaxiality between the valve during release and the native valve, improving the stability and accuracy during the release of the valve. However, the current valve delivery system has the problem of low control precision of bending adjustment.

### SUMMARY

The present disclosure provides a valve delivery device to solve the problem of low control precision of bending adjustment of the valve delivery device.

In embodiments of the present disclosure, a valve delivery device is provided. The valve delivery device includes: a bending adjustment tube; a withdrawing assembly connected to the bending adjustment tube and configured to install a valve; a bending adjustment wheel provided with a traction block; a traction wire, including an end connected to the withdrawing assembly and another end connected to the traction block; and a stabilizing tube that sleeves the bending adjustment tube and is arranged between the withdrawing assembly and the bending adjustment wheel. The bending adjustment wheel is configured to adjust a position of the traction block; the traction block drives the withdrawing assembly to move by means of the traction wire, to adjust bending degree of the bending adjustment tube; and the stabilizing tube is configured to limit a bending position of the bending adjustment tube.

The valve delivery device provided by the embodiments of the present disclosure is used to deliver the valve into the human body and install it at an appropriate position. The bending adjustment tube is used to guide the valve delivery device into the body. The withdrawing assembly sleeves the bending adjustment tube and connected to the traction wire. The withdrawing assembly and the traction wire may not be arranged at a same axis (for example, be eccentrically arranged). When the traction wire pulls the withdrawing assembly by the movement of the traction block, a direction of the applied force forms an angle with the axial direction of the withdrawing assembly, thereby being convenient for pulling the withdrawing assembly to bend the bending adjustment tube, and driving the outer tube for storing the valve to bend synchronously, so that the valve delivery device has better coaxiality with the physiological valve, thereby improving the precision of the valve release. The valve delivery device may enter the heart through blood vessels. Commonly, it may enter through the femoral artery, femoral vein, and superior vena cava. There is a certain distance between a position where the valve delivery device enters from the body surface and a target position, and a path for the valve delivery device to enter the target position is either straight or curved. The stabilizing tube sleeves the bending adjustment tube, and the hardness of the stabilizing tube is greater than that of the bending adjustment tube. When the bending adjustment tube is bent by being pulled by the traction wire, the stabilizing tube can limit the bending degree of the bending adjustment tube at a region where the stabilizing tube covers the bending adjustment tube, thereby limiting the bending position of the bending adjustment tube, so that a part of the bending adjustment tube that is not covered by the stabilizing tube is bent, making the adjustment of the bending adjustment tube by the traction wire more precise, which better adapts to the bending shape of the physiological tube wall of the human body.

In a possible implementation manner, the valve delivery device includes a bending adjustment tube base, the bending adjustment tube base is connected to the bending adjustment tube and configured to fix the bending adjustment tube.

The bending adjustment tube is connected to the bending adjustment base. The bending adjustment base is axially fixed to the bending adjustment wheel. When the valve delivery device enters a human body, a part of the bending adjustment tube outside the body can be fixed. When the bending adjustment wheel adjusts the bending degree of the bending adjustment tube, the bending adjustment tube does not move in the axial direction of the valve delivery device, thereby improving the precision of adjusting the bending adjustment tube.

In a possible implementation manner, the bending adjustment wheel is provided with internal threads, the traction block is provided with external threads, and the bending adjustment wheel sleeves the traction block and configured to drive the traction block to move along an axial direction of the bending adjustment tube when the bending adjustment wheel rotates.

The internal threads of the bending adjustment wheel and the external threads of the traction block are engaged with each other. When the bending adjustment wheel rotates, the traction block is driven to move along the axial direction of the valve delivery device. The traction block drives the traction wire to achieve the adjustment of the bending adjustment tube. The bending adjustment wheel and the traction block are connected through threads, the rotation of the bending adjustment wheel can be converted to be the translational movement of the traction block, thereby improving the control precision of the traction block, and thus accurately bending the bending adjustment tube.

In a possible implementation manner, the withdrawing assembly includes a withdrawing base provided with a first connection hole and a second connection hole, the first connection hole is arranged at an axis of the withdrawing base and configured for connecting the bending adjustment tube, and the second connection hole is configured for connecting the traction wire.

The first connection hole is arranged at the axis of the withdrawing base. The bending adjustment tube is connected to the withdrawing base by means of the first connection hole, so that the withdrawing base is at the axis of the bending adjustment tube. The second connection hole is used to for connecting the traction wire. When the traction wire is pulled, the traction wire drives the withdrawing base to move, thereby adjusting the bending degree of the bending adjustment tube.

In a possible implementation manner, the valve delivery device includes a bolt wire assembly including an bolt wire and an bolt wire, the bolt wire is installed at the bolt wire base, and the bolt wire assembly is arranged in the withdrawing base and is able to slide along an inner wall of the withdrawing base.

The bolt wire is used to cooperate with the valve. The valve may be provided with a fixing hole. The bolt wire can pass through the fixing hole to make the valve in a connected state. The withdrawing base sleeves the bolt wire base. The bolt wire base is arranged in the withdrawing base and can slide along the withdrawing base. The withdrawing base plays a guiding role in the movement of the bolt wire base. When the bolt wire base moves along the axial direction of the valve delivery device and the bolt wire is separated from the valve, the valve can be released and expanded.

In a possible implementation manner, the withdrawing base is provided with a guide groove, and the bolt wire base is provided with a guide boss. The guide boss is able to slide along the guide groove, and the bolt wire is arranged in the guide boss.

The inner wall of the withdrawing base is provided with a guide groove. The side wall of the bolt wire assembly is provided with a guide boss. The guide boss can slide along the guide groove, so as to limit the rotation of the bolt wire base relative to the withdrawing base, thereby reducing the possibility of the bending adjustment tube or the traction wire being twisted.

In a possible implementation manner, the bolt wire base is provided with a plurality of guide bosses distributed along a circumferential direction of the bolt wire base. The bolt wire is arranged corresponding to the guide boss.

The bolt wire base is provided with a plurality of guide bosses along its own circumferential direction, to limit the bolt wire base in different directions, thereby improving the stability of the bolt wire assembly when it moves. For example, the bolt wire assembly may be provided with three guide bosses evenly arranged along its own circumferential direction.

In a possible implementation manner, the withdrawing assembly includes a fixing claw, the fixing claw is installed at the withdrawing base, and the fixing claw and the guide groove are arranged at a same straight line in a radial direction of the withdrawing base.

The withdrawing assembly is provided with a fixing claw. The fixing claw may be provided with a limiting hole. At least a part of the valve can pass through the limiting hole and then the valve is fixed by means of the bolt wire, to limit the valve from being separated from the limiting hole. When releasing the valve, the fixing claw can limit the valve from being fully expanded, and realize partial expansion of the valve to achieve an effect of delayed release, thereby reducing the possibility of valve displacement or damage to the human tube wall caused by large deformation during the valve being directly released. The fixing claw and the guide groove are arranged correspondingly, thereby being convenient for the bolt wire of the guide boss and the fixing claw to cooperate with each other to fix the valve.

In a possible implementation manner, the valve delivery device includes a wire guide tube, and the wire guide tube includes an end connected to the bolt wire assembly, and another end provided with a locking device for limiting movement of the wire guide tube.

The wire guide tube is connected to the bolt wire assembly to control the movement of the bolt wire assembly. The other end of the wire guide tube is provided with a locking device. The locking device may include a locking member, an insurance groove provided at a tail of the valve delivery device, and a locking groove provided at a tail of the wire guide tube. The insurance groove and the locking groove may be in communication with each other. The limiting member may extend into the locking groove through the insurance groove, thereby limiting the movement of the wire guide tube. When the limiting member is separated from the locking hole, the wire guide tube can move. By limiting the wire guide tube by means of the locking device, the possibility of accidentally operating and moving the wire guide tube can be reduced, and the reliability of the valve delivery device can be improved.

In a possible implementation manner, the valve delivery device includes an outer tube and a release wheel, the outer tube sleeves the withdrawing assembly and the bending adjustment tube, the outer tube is configured to store the valve, and the release wheel is configured to control linear movement of the outer tube.

The outer tube is configured to store the valve. The release wheel is connected to the outer tube. The release wheel can rotate about the axis of the valve delivery device to control the movement of the outer tube, thereby improving the control precision of the outer tube. The outer tube and the withdrawing assembly cooperate with each other to control the release of the valve, thereby improving the accuracy of valve release.

The present disclosure provides a valve delivery device. The valve delivery device includes a bending adjustment tube, a withdrawing assembly, a traction wire, a bending adjustment wheel and a stabilizing tube. The withdrawing assembly is connected to the bending adjustment tube. The bending adjustment wheel is provided with a traction block. An end of the traction wire is connected to the withdrawing assembly, and another end of the traction wire is connected to the traction block. The stabilizing tube sleeves the bending adjustment tube and is arranged between the withdrawing assembly and the bending adjustment wheel. The bending adjustment wheel is configured to adjust the position of the traction block. The traction block drives, by mean of the traction wire, the withdrawing assembly to move to adjust the bending degree of the bending adjustment tube. The stabilizing tube is configured to limit the bending position of the bending adjustment tube, thereby improving the control precision of the bending shape of the valve delivery device.

It should be understood that the above general description and the following detailed description are only exemplary and cannot limit the present disclosure.

### BRIEF DESCRIPTION OF DRAWINGS

Fig. 1 is a schematic structural diagram of a valve delivery device provided by the present disclosure;
Fig. 2 is a partial cross-sectional view of a valve delivery device provided by the present disclosure;
Fig. 3 is a schematic structural diagram of a bolt wire assembly provided by the present disclosure;
Fig. 4 is a schematic structural diagram of a withdrawing assembly provided by the present disclosure; and
Fig. 5 is a schematic structural diagram of a withdrawing assembly from another perspective.

### Reference signs:

1 - bending adjustment tube;
11 - bending adjustment tube base;
2 - withdrawing assembly;
21 - withdrawing base;
211 - first connection hole;
212 - second connection hole;
213 - guide groove;
22 - fixing claw;
3 - bending adjustment wheel;
31 - traction block;
4 - traction wire;
5 - stabilizing tube;
6 - bolt wire assembly;
61 - bolt wire;
62 - guide boss;
63 - bolt wire base;
7 - wire guide tube;
71 - locking device;
8 - outer tube;
9 - release wheel.

The accompanying drawings herein are incorporated into the description and form a part of this description, showing embodiments in accordance with the present disclosure and are used together with the description to explain the principles of the present disclosure.

### DESCRIPTION OF EMBODIMENTS

To better illustrate the technical solutions of the present disclosure, the following will describe the embodiments of the present disclosure in conjunction with the accompanying drawings in detail.

It should be clear that the described embodiments are only some embodiments of the present disclosure, not all embodiments of the present disclosure. Based on the embodiments in the present disclosure, all other embodiments obtained by those of ordinary skill in the art without creative efforts shall fall within the protection scope of the present disclosure.

The terms used in the embodiments of the present disclosure are merely for the purpose of describing particular embodiments but not intended to limit the present disclosure. Unless otherwise noted in the context, the singular form expressions "a", "an", "the" and "said" used in the embodiments and appended claims of the present disclosure are also intended to represent plural form expressions thereof.

It should be understood that the term "and/or" used herein is merely an association relationship describing associated objects, indicating that there may be three relationships, for example, A and/or B may indicate that three cases, i.e., A existing individually, A and B existing simultaneously, B existing individually. In addition, the character "/" herein generally indicates that the related objects before and after the character form an "or" relationship.

It should be noted that, the expressions such as "upper", "lower", "left", "right" and the like mentioned in embodiments of the present disclosure are described with reference to the placement status in the accompanying drawings, and should not be construed as limiting embodiments of the present disclosure. In addition, it should also be understood that, in the context, while referring to an element being formed "above" or "below" another element, it is possible that the element is directly formed "above" or "below" the other element, it is also possible that the element is formed "above" or "below" the other element via an intermediate element.

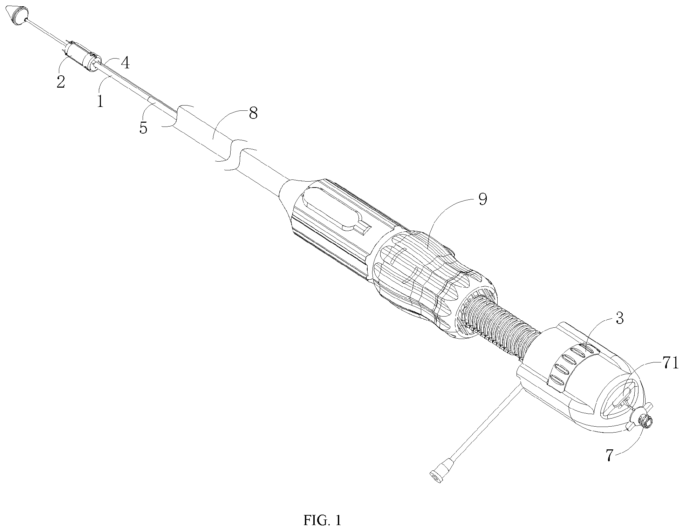

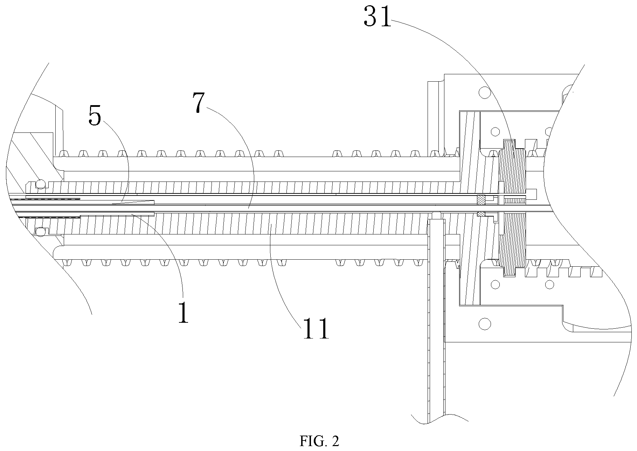

As shown in Figs. 1 and 2, the embodiments of the present disclosure provide a valve delivery device. The valve delivery device includes a bending adjustment tube 1, a withdrawing assembly 2, a traction wire 4, a bending adjustment wheel 3 and a stabilizing tube 5. The withdrawing assembly 2 is connected to the bending adjustment tube 1. The withdrawing assembly 2 is used for installing a valve. The bending adjustment wheel 3 is provided with a traction block 31. An end of the traction wire 4 is connected to the withdrawing assembly 2, and another end of the traction wire 4 is connected to the traction block 31. The stabilizing tube 5 sleeves the bending adjustment tube 1 and is arranged between the withdrawing assembly 2 and the bending adjustment wheel 3. The bending adjustment wheel 3 is used to adjust a position of the traction block 31. The traction block 31 drives the withdrawing assembly 2 to move through the traction wire 4, so as to adjust a bending degree of the bending adjustment tube 1. The stabilizing tube 5 is used to limit a bending position of the bending adjustment tube 1.

The valve delivery device provided by the embodiments of the present disclosure is used to deliver the valve into a human body, and install and release the valve at an appropriate position. The bending adjustment tube 1 is used to guide the valve delivery device into the human body. The withdrawing assembly 2 sleeves the bending adjustment tube 1 and is connected to the traction wire 4. The withdrawing assembly 2 and the traction wire 4 may not be arranged at a same axis (for example, be eccentrically arranged). When the traction wire 4 pulls the withdrawing assembly 2 by the movement of the traction block 31, a direction of the applied force forms an angle with the axial direction of the withdrawing assembly 2, thereby being convenient for pulling the withdrawing assembly 2 to bend the bending adjustment tube 1, and driving the outer tube for storing the valve to bend synchronously, so that the valve delivery device has better coaxiality with the physiological valve, thereby improving the precision of the valve release. The valve delivery device may enter the heart through blood vessels. Commonly, it may enter through the femoral artery, femoral vein, and superior vena cava. There is a certain distance between a position where the valve delivery device enters from the body surface and a target position, and a path for the valve delivery device to enter the target position is either straight or curved. The stabilizing tube 5 sleeves the bending adjustment tube 1, and the hardness of the stabilizing tube 5 is greater than that of the bending adjustment tube 1. When the bending adjustment tube 1 is bent by being pulled by the traction wire 4, the stabilizing tube 5 can limit the bending degree of the bending adjustment tube 1 at a region where the stabilizing tube 5 covers the bending adjustment tube 1, thereby limiting the bending position of the bending adjustment tube 1, so that a part of the bending adjustment tube 1 that is not covered by the stabilizing tube 5 is bent, making the adjustment of the bending adjustment tube 1 by the traction wire 4 more precise, which better adapts to the bending shape of the physiological tube wall of the human body.

As shown in Fig. 2, in a possible implementation manner, the valve delivery device includes a bending adjustment tube base 11, the bending adjustment tube base 11 is connected to the bending adjustment tube 1 and is used to fix the bending adjustment tube 1.

The bending adjustment tube 1 is connected to the bending adjustment base. The bending adjustment base is axially fixed to the bending adjustment wheel 3. When the valve delivery device enters the human body, the bending adjustment tube 1 can be relatively fixed. When the bending adjustment wheel 3 adjusts the bending degree of the bending adjustment tube 1, the bending adjustment tube 1 does not move in the axial direction of the valve delivery device, improving the precision of adjusting the bending adjustment tube 1.

As shown in Fig. 2, in a possible implementation manner, the bending adjustment wheel 3 is provided with internal threads, and the traction block 31 is provided with external threads. The bending adjustment wheel 3 sleeves the traction block 31 and is used to drive the traction block 31 to move along an axial direction of the bending adjustment tube 1 when the bending adjustment wheel 3 rotates.

The internal threads of the bending adjustment wheel 3 and the external threads of the traction block 31 are engaged with each other. When the bending adjustment wheel 3 rotates, the traction block 31 is driven to move along the axial direction of the valve delivery device. The traction block 31 drives the traction wire 4, to achieve the adjustment of the bending adjustment tube 1. By means of the engagement between the bending adjustment wheel 3 and the traction block 31 through threads, the rotation of the bending adjustment wheel 3 can be converted to be the translational movement of the traction block 31, thereby improving the control precision of the traction block 31, and thus accurately bending the bending adjustment tube 1.

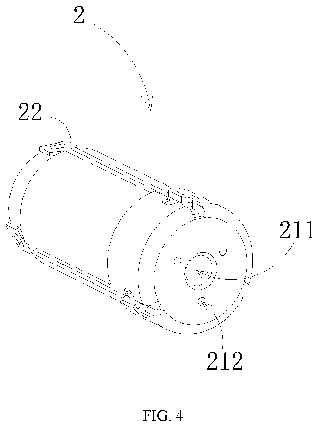

As shown in Fig. 4, in a possible implementation manner, the withdrawing assembly 2 includes a withdrawing base 21, the withdrawing base 21 is provided with a first connection hole 211 and a second connection hole 212, the first connection hole 211 is arranged at the axis of the withdrawing base 21 and is used for connecting the bending adjustment tube 1, and the second connection hole 212 is used for connecting the traction wire 4.

The first connection hole 211 is arranged at the axis of the withdrawing base 21. The bending adjustment tube 1 is connected to the withdrawing base 21 through the first connection hole 211, so that the withdrawing base 21 is at the axis of the bending adjustment tube 1. The second connection hole 212 is used for connecting the traction wire 4. When the traction wire 4 is pulled, the traction wire 4 drives the withdrawing base 21 to move, thereby adjusting the bending degree of the bending adjustment tube 1.

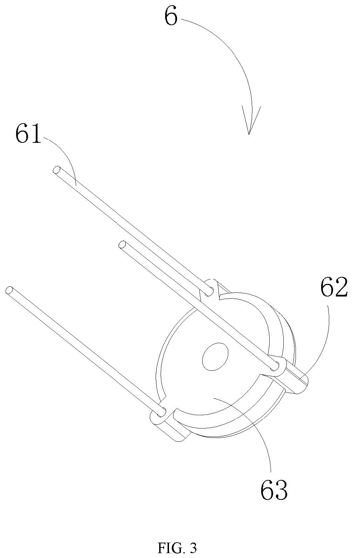

As shown in Fig. 3, in a possible implementation manner, the valve delivery device includes a bolt wire assembly 6, the bolt wire assembly 6 includes an bolt wire 61 and an bolt wire base 63, the bolt wire 61 is installed at the bolt wire base 63, and the bolt wire assembly 6 is arranged in the withdrawing base 21 and is able to slide along an inner wall of the withdrawing base 21.

The bolt wire 61 is used to cooperate with the valve. The valve may be provided with a fixing hole. The bolt wire 61 can pass through the fixing hole to make the valve in a connected state. The withdrawing base 21 sleeves the bolt wire base 63. The bolt wire base 63 is arranged in the withdrawing base 21 and can slide along the withdrawing base 21. The withdrawing base 21 plays a guiding role in the movement of the bolt wire base 63. When the bolt wire base 63 moves along the axial direction of the valve delivery device and the bolt wire 61 is separated from the valve, the valve can be released and expanded.

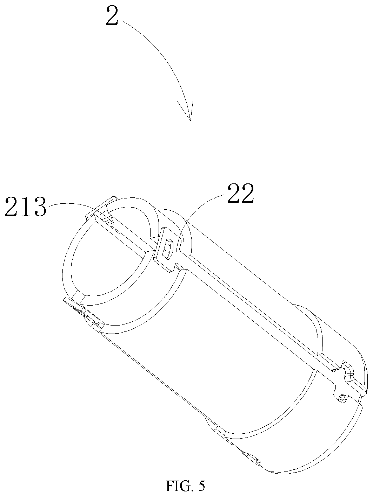

As shown in Figs. 3 and 5, in a possible implementation manner, the withdrawing base 21 is provided with a guide groove 213, and the bolt wire base 63 is provided with a guide boss 62, the guide boss 62 is able to slide along the guide groove 213, and the bolt wire 61 is arranged at the guide boss 62.

The inner wall of the withdrawing base 21 is provided with a guide groove 213. The side wall of the bolt wire assembly 6 is provided with a guide boss 62. The guide boss 62 can slide along the guide groove 213, thereby limiting the rotation of the bolt wire base 63 relative to the withdrawing base 21, and reducing the possibility of the bending adjustment tube 1 or the traction wire 4 being twisted.

As shown in Fig. 3, in a possible implementation manner, the bolt wire base 63 is provided with a plurality of guide bosses 62 distributed along a circumferential direction of the bolt wire base 63. The bolt wire 61 is arranged corresponding to the guide boss 62.

The bolt wire base 63 is provided with a plurality of guide bosses 62 distributed along its own circumferential direction, thereby limiting the bolt wire base 63 in different directions and improving the stability of the bolt wire assembly 6 when it moves. For example, the bolt wire assembly 6 may be provided with three guide bosses 62 evenly distributed along its own circumferential direction.

As shown in Fig. 4, in a possible implementation manner, the withdrawing assembly 2 includes a fixing claw 22 fixedly installed at the withdrawing base 21, and the fixing claw 22 and the guide groove 213 are arranged at a same straight line in a radial direction of the withdrawing base 21.

The withdrawing assembly 2 is provided with a fixing claw 22. The fixing claw 22 may be provided with a limiting hole. At least a part of the valve can pass through the limiting hole and then the valve is fixed by the bolt wire 61 to limit the valve from being separated from the limiting hole. When releasing the valve, the fixing claw 22 can limit the valve from being fully expanded, and realize partial expansion of the valve to achieve an effect of delayed release, thereby reducing the possibility of valve displacement or damage to the human tube wall caused by large deformation during the valve being directly released. The fixing claw 22 and the guide groove 213 are arranged correspondingly, thereby being convenient for the bolt wire 61 of the guide boss 62 and the fixing claw 22 to cooperate with each other to fix the valve.

As shown in Figs. 1 and 2, in a possible implementation manner, the valve delivery device includes a wire guide tube 7, an end of the wire guide tube 7 is connected to the bolt wire assembly 6, and another end of the wire guide tube 7 is provided with a locking device 71, for limiting the movement of the wire guide tube 7.

The wire guide tube 7 is connected to the bolt wire assembly 6 to control the movement of the bolt wire assembly 6. Another end of the wire guide tube 7 is provided with a locking device 71. The locking device 71 may include a locking member, an insurance groove provided at a tail of the valve delivery device, and a locking groove provided at a tail of the wire guide tube 7. The insurance groove and the locking groove may be in communication with each other. The limiting member may extend into the locking groove through the insurance groove, thereby limiting the movement of the wire guide tube 7. When the limiting member is separated from the locking hole, the wire guide tube 7 can move. By limiting the wire guide tube 7 by means of the locking device 71, the possibility of accidentally operating and moving the wire guide tube 7 can be reduced, and the reliability of the valve delivery device can be improved.

As shown in Fig. 1, in a possible implementation manner, the valve delivery device includes an outer tube 8 and a release wheel 9, the outer tube 8 sleeves the withdrawing assembly 2 and the bending adjustment tube 1 and is used to store the valve, and the release wheel 9 is used to control the linear movement of the outer tube 8.

The outer tube 8 is used to store the valve. The release wheel 9 is connected to the outer tube 8. The release wheel 9 can rotate about the axis of the valve delivery device to control the movement of the outer tube 8, improving the control precision of the outer tube 8. The outer tube 8 and the withdrawing assembly 2 cooperate with each other to control the release of the valve, thereby improving the accuracy of valve release.

The embodiments of the present disclosure provide a valve delivery device. The valve delivery device includes a bending adjustment tube 1, a withdrawing assembly 2, a traction wire 4, a bending adjustment wheel 3 and a stabilizing tube 5. The withdrawing assembly 2 is connected to the bending adjustment tube 1. The bending adjustment wheel 3 is provided with a traction block 31. An end of the traction wire 4 is connected to the withdrawing assembly 2, and another end of the traction wire 4 is connected to the traction block 31. The stabilizing tube 5 sleeves the bending adjustment tube 1 and is arranged between the withdrawing assembly 2 and the bending adjustment wheel 3. The bending adjustment wheel 3 is used to adjust the position of the traction block 31. The traction block 31 drives the withdrawing assembly 2 to move through the traction wire 4, to adjust the bending degree of the bending adjustment tube 1. The stabilizing tube 5 is used to limit the bending position of the bending adjustment tube, thereby improving the control precision of the bending shape of the valve delivery device.

The above-described embodiments are merely preferred embodiments of the present disclosure and are not intended to limit the present disclosure. Various changes and modifications can be made to the present disclosure by those skilled in the art. Any modifications, equivalent substitutions and improvements made within the principle of the present disclosure shall fall into the protection scope of the present disclosure.

## Claims

1. A valve delivery device, comprising:
a bending adjustment tube (1);
a withdrawing assembly (2) connected to the bending adjustment tube (1) and configured to install a valve;
a bending adjustment wheel (3) provided with a traction block (31);
a traction wire (4), comprising an end connected to the withdrawing assembly (2) and another end connected to the traction block (31); and
a stabilizing tube (5) that sleeves the bending adjustment tube (1) and is arranged between the withdrawing assembly (2) and the bending adjustment wheel (3),
wherein the bending adjustment wheel (3) is configured to adjust a position of the traction block (31); the traction block (31) drives the withdrawing assembly (2) to move by means of the traction wire (4), to adjust a bending degree of the bending adjustment tube (1); and the stabilizing tube (5) is configured to limit a bending position of the bending adjustment tube (1).

2. The valve delivery device according to claim 1, further comprising a bending adjustment tube base (11) connected to the bending adjustment tube (1) and configured to fix the bending adjustment tube (1).

3. The valve delivery device according to claim 2, wherein the bending adjustment wheel (3) is provided with internal threads, the traction block (31) is provided with external threads, and the bending adjustment wheel (3) sleeves the traction block (31) and is configured to drive the traction block (31) to move along an axial direction of the bending adjustment tube (1) when the bending adjustment wheel (3) rotates.

4. The valve delivery device according to claim 1, wherein the withdrawing assembly (2) comprises a withdrawing base (21), the withdrawing base (21) is provided with a first connection hole (211) and a second connection hole (212), the first connection hole (211) is arranged at an axis of the withdrawing base (21) and is configured for connecting the bending adjustment tube (1), and the second connection hole (212) is configured for connecting the traction wire (4).

5. The valve delivery device according to claim 4, further comprising a bolt wire assembly (6), wherein the bolt wire assembly (6) comprises an bolt wire (61) and an bolt wire base (63), the bolt wire (61) is installed at the bolt wire base (63), the bolt wire assembly (6) is arranged in the withdrawing base (21) and is able to slide along an inner wall of the withdrawing base (21).

6. The valve delivery device according to claim 5, wherein the withdrawing base (21) is provided with a guide groove (213), the bolt wire base (63) is provided with a guide boss (62), the guide boss (62) is able to slide along the guide groove (213), and the bolt wire (61) is arranged in the guide boss (62).

7. The valve delivery device according to claim 6, wherein the bolt wire base (63) is provided with a plurality of guide bosses (62) distributed along a circumferential direction of the bolt wire base (63), and the bolt wire (61) is arranged corresponding to the guide boss (62).

8. The valve delivery device according to claim 6, wherein the withdrawing assembly (2) comprises a fixing claw (22), the fixing claw (22) is fixedly installed at the withdrawing base (21), and the fixing claw (22) and the guide groove (213) are arranged at a same straight line in a radial direction of the withdrawing base (21).

9. The valve delivery device according to claim 5, further comprising a wire guide tube (7), wherein the wire guide tube (7) comprises an end connected to the bolt wire assembly (6), and another end provided with a locking device (71) for limiting movement of the wire guide tube (7).

10. The valve delivery device according to any one of claims 1 to 9, further comprising an outer tube (8) and a release wheel (9), wherein the outer tube (8) sleeves the withdrawing assembly (2) and the bending adjustment tube (1), the outer tube (8) is configured to store the valve, and the release wheel (9) is configured to control linear movement of the outer tube (8).
